(19) Europäisches Patentamt
European Patent Office
Office européen des brevets

(11) Veröffentlichungsnummer : **0 406 259 B1**

(12) # EUROPÄISCHE PATENTSCHRIFT

(45) Veröffentlichungstag der Patentschrift : **16.06.93 Patentblatt 93/24**

(21) Anmeldenummer : **89903099.3**

(22) Anmeldetag : **08.03.89**

(86) Internationale Anmeldenummer : **PCT/DE89/00146**

(87) Internationale Veröffentlichungsnummer : **WO 89/08845 21.09.89 Gazette 89/23**

(51) Int. Cl.$^5$ : **G01N 33/574,** C07K 15/28, C12P 21/08, C12N 5/00, A61K 39/395, C07H 13/04, C08B 37/00, // (C12P21/00, C12R1:91)

(54) **ANTIKÖRPER, VERFAHREN ZU IHRER HERSTELLUNG, KLONE ZUR ERZEUGUNG DER ANTIKÖRPER SOWIE VERWENDUNG DER ANTIKÖRPER ZUR DIAGNOSE UND THERAPIE VON TUMOREN.**

(30) Priorität : **08.03.88 DE 3807594**

(43) Veröffentlichungstag der Anmeldung : **09.01.91 Patentblatt 91/02**

(45) Bekanntmachung des Hinweises auf die Patenterteilung : **16.06.93 Patentblatt 93/24**

(84) Benannte Vertragsstaaten : **AT BE CH DE FR GB IT LI LU NL SE**

(56) Entgegenhaltungen :
**EP-A- 0 199 196**
**Biochemistry, Band 24, Nr.17, 1985, The American Chemical Society (Easton, P.A., US), A.L. Tarentino et al."Deglycosylation of asparagine-linked glycans by peptides : N-glycosidase F", pages 4665-4671**
**The EMBO Journal, Band 5, Nr.9, 1986, IRL Press Ltd (Oxford, GB), R. Tauber et al."Different oligosaccharide processing of the membrane-integrated and the secretory form of gp 80 in rat liver", pages 2109-2114**

(73) Patentinhaber : **REUTTER, Werner**
**Arnimallee 22**
**W-1000 Berlin 33 (DE)**

(72) Erfinder : **REUTTER, Werner**
**Arnimallee 22**
**W-1000 Berlin 33 (DE)**
Erfinder : **NUCK, Rolf**
**Sodenerstrasse 34**
**W-1000 Berlin 33 (DE)**
Erfinder : **ZIMMERMANN, Martin**
**Dominicusstrasse 44**
**W-1000 Berlin 62 (DE)**

(74) Vertreter : **Ruschke, Hans Edvard, Dipl.-Ing. et al**
**Patentanwälte Ruschke & Partner**
**Pienzenauerstrasse 2**
**W-8000 München 80 (DE)**

EP 0 406 259 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue Antikörper, die Immunglobuline der Klassen G und M mit Mulekulargewichten von 100 000 bis 900 000 darstellen und gegen den Oligosaccharid-Teil von Glycoproteinen, d.h. gegen N-Glycanstrukturen von Zellmembranoberflächen, gerichtet sind und im Immun-Test (z.B. im RIA- und ELISA-Test oder im Mikrotiterplatten-Test) eine positive Reaktion gegen antigene Oligosaccharide mit antigenen Strukturelementen aus Chitobiose-Mannose-Trisacchariden der nachstehend angegebenen allgemeinen Formel (I) zeigen, Verfahren und Klone zu ihrer Herstellung sowie ihre Verwendung zur Herstellung eines Mittels für die Diagnose und Therapie von Tumoren, insbesondere Hepatomen und Dickdarmtumoren, da sie die organspezifischen Struktureigenheiten von tumorrelevanten Glycoproteinen erkennen und signalisieren können und in die Glycoproteinchemie der Zellmembranoberflächen von malignen Tumorgeweben bindend eingreifen.

Bisher werden Antikörper durch Immunisierung gegen ganze Zellen, gegen Membranfragmente oder gegen isolierte Membranglycoproteine bzw. -lipide erzeugt, wobei im letzteren Fall das Gesamtmolekül des Proteins als antigene Determinante dient.

Es ist bisher jedoch noch nicht versucht worden, Antikörper getrennt gegen den Protein- und den (abgetrennten) Kohlenhydratanteil von Glycoproteinen zu erzeugen und in Seren zu testen. Da in den Glycoproteinen der Proteinanteil gegenüber dem Kohlenhydratanteil als antigene Determinante überwiegt, sind die bisher gewonnenen Antikörper weitgehend gegen die Proteinstruktur gerichtet. Dies verhindert, daß sich gegen zelltypische Oligosaccharidstrukturen von Glycoproteinen überhaupt in nennenswertem Ausmaß Antikörper bilden. Infolgedessen ist es bisher auch nicht möglich, strukturelle Feinheiten und Abweichungen im Glykananteil zu verfolgen und nachzuweisen.

In der Tumordiagnostik werden seit längerer Zeit bestimmte Serumglycoproteine, in erster Linie Foetoprotein und carcinoembryonales Antigen, gemessen (vgl. G. Uhlenbruck und F. Dati, "Zur Labordiagnostik von Tumorerkrankungen" in "Diagnose & Labor" 36, 41-55 (1986)). Sie sind erst beim Vorliegen eines relativ weit fortgeschrittenen Tumors vermehrt im Serum nachweisbar, sie sind jedoch relativ unspezifisch. Größere Spezifität wird mit der Messung von menschlichem Choriongonadotropin (hCG) erzielt, das bei bestimmten, relativ selten vorkommenden Tumoren der Gonaden und des Uterus positiv ist (vgl. Henry S. Kaplan, L. Olsson und A. Ranbitschek, "Monoclonal Human Antibodies: A recent development with wide-ranging clinical potential, Monoclonal Antibodies" in "Clinical Medicins" herausgegeben von A.7. Mc Michael und J.W. Fabre, Academic Press, London, New York, S. 16-35 (1982)).

Es wurde auch bereits die Brauchbarkeit der Messung der Aktivität von Fucosyl-Transferasen für die Tumordiagnostik im Serum geprüft (vgl. C.H. Bauer, W.G. Reutter, K.P. Erhart, E. Köttgen und W. Gerok, "Decrease of Human Serum Fucosyltransferase as an Indicator of Successful Tumor Therapy", in "Science" 201, 1232-1233 (1978)). In ihrer Aussagefähigkeit lassen sie sich mit den bisherigen Tumormarkern durchaus vergleichen. Zudem erbrachten sie beim Schilddrüsencarcinom positive Werte, was mit den obengenannten Markern bisher nicht gelungen ist. Trotzdem wurde auch mit diesen Enzymen keine wesentliche Verbesserung der Tumordiagnostik erzielt.

Das Problem einer organspezifischen Diagnostik bedarf vermutlich einer grundsätzlich anderen Lösung. In jüngster Zeit hat sich die Anwendung monoclonaler Antikörper bei der Lymphomdiagnostik und -typisierung als sehr nützlich erwiesen (vgl. H. Stein und D.Y. Mason, "Immunological analysis of tissue sections in diagnosis of lymphoma" in "Recent Advantages in Haematology", Hrsg. A.V. Hoffbrand et al., Livingstone (1985)).

Aus "Biochemistry 1985", 24, S. 4665 - 4671, ist es bekannt, daß das Enzym PNGase F aus einem Serumglycoprotein ein intaktes Oligosaccharid, d.h. eine N-Glycanstruktur eines Glycopeptids, die aus Strukturelementen aus Chitobiose-Mannose-Trisacchariden besteht, abspaltet, die Verwendung solcher intakter Oligosaccharide als Antigene zur Erzeugung von Antikörpern, die gegen N-Glycanstrukturen von Zellmembranoberflächen gerichtet sind, ist darin jedoch nicht erwähnt, abgesehen davon, daß in der auf Seite 4669 angegebenen Oligosaccharidstruktur der Rest N-N- unsubstituiert ist.

Aufgabe der Erfindung war es daher, neue spezifische Antikörper bereitzustellen, mit deren Hilfe Glycoproteine und Glycoproteinmuster spezifischer als bisher nachgewiesen werden können, um Zellen und Zellveränderungen, insbesondere bei Tumoren und Metastasen, organspezifisch diagnostizieren und behandeln zu können.

Glycoproteine sind bekanntlich essentielle Bausteine von Plasmamembranen, weil sie

a) zu 60 % die Plasmamembran bilden (z.B. in der Leber),

b) den Kontakt der Zelle zum äußeren Milieu und zu den Nachbarzellen vermitteln,

c) Informationen von außerhalb der Zelle zu erkennen und in das Zellinnere zu transferieren vermögen.

Das Glycoproteinmuster der Plasmamembran ist bei maligner Transformation verändert (vgl. P. Vischer und W. Reutter, "Specific Alterations of Fucoprotein Biosynthesis in the Plasma Membrane of Morris Hepatoma 7777" in "Eur. J. Biochem.", 84, 363-368 (1978), und T. Galleotti, A. Cittadini, G. Neri, S. Papa und L.A. Smits,

"Cell Membranes and Cancer", Elsevier, Amsterdam (1985)). Vermutlich ist auch die Struktur einzelner Glycoproteine nach maligner Transformation modifiziert (vgl. T. Galleotti, A. Cittadini, G. Neri, S. Papa und L.A. Smits (1985) "Cell Membranes and Cancer", Elsevier, Amsterdam (1985)). Diese Veränderungen sind von einer gestörten Informationsvermittlung, einem unregulierten Wachstum und schließlich dem Ablösen einiger Zellen aus dem Gewebeverband (Metastasierung) begleitet.

Als Veränderungen der Glycoprotein-Zusammensetzung konnten festgestellt werden:

a) das Verschwinden leberspezifischer Glycoproteine,

b) das Auftreten neuer Glycoproteine,

c) die Veränderung proteingleicher Glycoproteine in ihrem Kohlenhydratanteil.

Eine Veränderung des Glycoproteinmusters, wie es von einer normalen Zelle her bekannt ist, läßt somit auf eine maligne Entartung schließen. Die Diagnostik von Tumoren im allgemeinen und von Hepatocarcinomen im besonderen ist derzeit jedoch noch unbefriedigend.

Es wurde nun überraschend gefunden, daß der in den Zellmembran-Glycoproteinen enthaltene Kohlenhydrat-Anteil, wenn er von dem Proteinverband abgelöst wird, eine außerordentlich spezifische antigene Determinante für die Erzeugung von Antikörpern darstellt.

Gegenstand der Erfindung sind Antikörper, die dadurch gekennzeichnet sind, daß sie Immunoglobuline der Klassen G und M mit Molekulargewichten von 100 000 bis 900 000 darstellen und gegen N-Glycanstrukturen von Zellmembranoberflächen gerichtet sind und im Immuntest (z.B. im RIA- und ELISA-Test oder im Mikrotiterplattentest) eine positive Reaktion gegen antigene Oligosaccharide mit antigenen Strukturelementen aus Chitobiose-Mannose-Trisacchariden der allgemeinen Formel (I) zeigen:

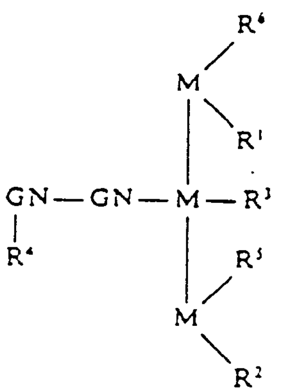

(I)

worin bedeuten:

| | |
|---|---|
| GN | N-Acetylglucosamin, |
| M | Mannose, wobei die Mannosereste über 1,3- und/oder 1,6-glycosidische Bindungen miteinander verknüpft sind und der Mannose-Rest am Chitobiose-Rest β-1,4-glycosidisch gebunden ist, |
| $R^1$, $R^2$, $R^5$ und $R^6$, | die gleich oder verschieden sind, jeweils Wasserstoff, den N-Acetylglucosamin-, Mannose-, Mannosedisaccharid- oder Lactosylamin-Rest GN-β-1,4-Gal, worin Gal für Galactose steht und GN und/oder Gal gegebenenfalls durch einen L-Fucosyl-Rest α-1,3-glycosidisch oder α-1,6-glycosidisch oder durch einen N-Acetylneuraminsäurerest vorzugsweise α-2,3-, α-2,6- oder α-2,8-glycosidisch substituiert ist (sind), |
| $R^3$ | Wasserstoff oder einen β-1,4-glycosidisch gebundenen und gegebenenfalls durch L-Fucose substituierten N-Acetylglucosamin- oder Lactosylamin-Rest GN-β-1,4-Gal, und |
| $R^4$ | Wasserstoff oder einen α-1,6-glycosidisch gebundenen L-Fucoserest. |

Die erfindungsgemäßen Antikörper sind spezifisch gegen N-Glycanstrukturen von Zellmembranoberflächen gerichtet. Sie zeigen im RIA- und ELISA-Test oder im Mikrotiterplatten-Test eine positive Reaktion gegen antigene Oligosaccharide mit Strukturelementen aus Chitobiose-Mannose-Trisacchariden der vorstehend angegebenen allgemeinen Formel (I) und sind daher geeignet zum Nachweis definierter, tumorspezifischer Membranproteine bzw. Membranproteinmuster im Serum von Tumorpatienten, insbesondere solchen mit Hepatomen.

Nach ihrer chemischen Struktur sind die erfindungsgemäßen Antikörper Immunglobuline der Klassen G und M mit einem Molekulargewicht im Bereich von 100 000 bis 900 000. Ihr Wirkungsoptimum liegt im Temperaturbereich von 20 bis 40°C.

Bevorzugte Ausführungsformen der erfindungsgemäßen Antikörper ergeben sich aus den Unteranspru-

chen 2 bis 6.

Gegenstand der Erfindung ist ferner ein Verfahren zur Herstellung der vorstehend beschriebenen Antikörper, das dadurch gekennzeichnet ist, daß man die Oligosaccharid-Fraktionen aus den Glycoproteinen der Zellmembranoberflächen abtrennt und immunologisch zur Erzeugung der Antikörper verwendet, sie insbesondere abspaltet und isoliert, die Oligosaccharide als Haptene koppelt, die Antigene einem Versuchstier, vorzugsweise Balb/c-Mäusen, injiziert, die Milzzellen des immunisierten Versuchstiers gewinnt und mit Myelom- oder Plasmacytom-Zellen fusioniert und die Antikörper aus dem Medium der reklonierten positiven Klone gewinnt, wobei vorzugsweise eine Suspension der Antigene in die Rückenhaut des Versuchstieres injiziert wird und die Antikörper vorzugsweise aus der Serumfraktion des Blutes des Versuchstieres gewonnen werden.

Weitere bevorzugte Ausgestaltungen des erfindungsgemäßen Verfahrens ergeben sich aus den Unteransprüchen 10 bis 13.

Gegenstand der Erfindung ist ferner ein Klon zur Erzeugung der vorstehend beschriebenen Antikörper gegen N-Glycanstrukturen von Zellmembranoberflächen, der dadurch gekennzeichnet ist, daß er durch Fusionieren von Myelom- oder Plasmocytom-Zellen mit den Milzzellen eines Versuchstieres herstellbar ist, das mit Oligosaccharid-Antigenen immunisiert wurde, die aus der Oligosaccharid-Fraktion von Glycoproteinen der Zellmembranoberflächen gebildet wurden.

Die Erfindung betrifft schließlich auch die Verwendung der vorstehend beschriebenen Antikörper zur Herstellung eines Mittels für die Diagnose und Therapie von Tumoren, insbesondere Hepatomen und Dickdarmtumoren.

Die Erfindung wird nachstehend näher erläutert.

Die Antikörper der Erfindung werden allgemein hergestellt, indem man die Oligosaccharid-Fraktion aus Glycoproteinen der Zellmembranoberflächen abspaltet und nach Koppeln der Haptene immunologisch als Antigene zur Erzeugung der Antikörper verwendet. Die Abspaltung des Oligosaccharid-Anteils geschieht entweder chemisch, z.B. durch Hydrazinolyse, oder enzymatisch unter Verwendung geeigneter Enzyme wie Amidase F (E.C. 3.2.2.18), Endoglycosidase F (E.C. 3.2.1.96) und H (E.C. 3.2.1.96).

Die Oligosaccharid-Fraktion wird zunächst gereinigt und gegebenenfalls in die verschiedenen Oligosaccharidkörper aufgetrennt. Die isolierten Oligosaccharide werden dann als Haptene an einen geeigneten Träger, z.B. an Albumin oder Edestin, chemisch gekopplet und als Antigene zur Erzeugung der Antikörper verwendet.

Die Antikörpererzeugung kann polyklonal oder monoklonal erfolgen.

Als geeigente Träger zur Kopplung der Haptene eignen sich insbesondere Albumin und Edestin.

Die polyklonale Erzeugung der Antikörper erfolgt vorzugsweise durch Injizieren einer micellaren Antigensuspension in die Rückenhaut eines Versuchstieres und Gewinnen der Antikörper aus der Serumfraktion des Blutes des Versuchstieres. Als Versuchstiere dienen insbesondere Kaninchen oder Hühner.

Die monoklonale Erzeugung der Antikörper wird vorzugsweise so ausgeführt, daß die zu einem Antigen gekoppelten Haptene einem Versuchstier injiziert werden, wobei insbesondere verschiedene Mäusestämme wie Balb/c, NSI, NSO oder Rattenstämme wie $Y_3$, YB2/0 in Betracht kommen. Dann werden die Milzzellen des immunisierten Tieres gewonnen und mit Myelom- oder Plasmocytom-Zellen von Balb/c-Mäusen, z.B. X.63/Ag8653, fusioniert und die Antikörper aus dem Medium der reklonierten positiven Klone gewonnen.

Diese Klone sind ebenfalls Gegenstand der Erfindung.

Die Strukturvielfalt und der Verästelungsgrad der Oligosaccharide in den Glycoproteinen sind wesentlich größer als die der linearen Proteine. Es war deshalb überraschend, daß die Antikörper in hochdifferenzierter Form und sogar monospezifisch erhalten werden können, wodurch eine Gruppe von immunologischen Struktursensoren geschaffen wurde, die nicht nur die Struktur der normalen Glycoproteine differenziert erkennen können, sondern auch die in ihrem Kohlenhydratanteil abgewandelten Glycoproteine, so wie sie von Tumorzellen gebildet werden, zu diagnostizieren vermögen.

In diesem Zusammenhang wurden auch die Oligosaccharide der Gly-coproteine von normalen und malignen Zellmembranen analysiert und aufgeklärt. Allen N-Glycan-Oligosacchariden liegt ein Chitobiose-Mannose-Trisaccharid zugrunde, das in unterschiedlichen Mengen und Positionen durch L-Fucose, D-Mannose, N-Acetyl-D-glucosamin, Lactosylamin und N-Acetylneuraminsäure substituiert ist. Die Anzahl der Monosaccharideinheiten liegt bei 6 bis 24. Sehr charakteristisch ist der hohe Fucose- und Mannoseanteil, der im Falle von reifen Tumorzellen festzustellen ist.

Die Wechselbeziehung von Strukturantigenität und Antikörperaktivität ist auf dem Gebiet der Oligosaccharide mit Strukturen der Formel (I) wesentlich besser differenzierbar und deshalb sensibler als bei Proteinen. Hierdurch wird ein Weg zur organspezifischen Tumordiagnose eröffnet.

Mit den erfindungsgemäßen Antikörpern ist die Möglichkeit gegeben, die peripheren Bereiche einer Zelle, die mit dem umgebenden Milieu und den Nachbarzellen kommunizieren, zu erreichen; sie brauche nicht zum Proteinanteil vorzudringen, der viel tiefer in der Zelloberfläche (Lipiddoppelschicht) veraankert ist.

Dieses neue diagnostische Vorgehen bietet die folgenden Vorteile:

- es werden Antikörper erzeugt gegen Oberflächenstrukturen, die besser zugänglich sind als die in die Membran eingelagerten Proteinträger;
- die Zell- bzw. Organspezifität wid vornehmlich durch die Oligosaccharidstruktur und -zusammensetzung der Zellmembranen geprägt.

Die Antikörper werden identifiziert durch einen ELISA-Test (Enzym Linked ImmunoSorbent Assay). Hierzu wird das Antigen (Zell- bzw. Membranextrakte) auf eine Kunststoffoberfläche aufgebracht (z.B. in die Vertiefungen von 96-Well-Mikrotiter-Platten), antrocknen lassen (30 min), danach mit Maus-IgG's (= die zu prüfenden Antikörper) inkubiert; dreimal mit einem Puffer gewaschen (PBS = Phosphat gepufferte Kochsalzlösung); dann wird mit Anti-Maus-IgG's vom Schaf, die mit einem Enzym, z.B. Peroxidase, gekoppelt sind, inkubiert (2 h bei Raumtemperatur), und mit PBS gewaschen. Nach Zugabe des Substras Orthophenylendiamin (200 µ) und Abstoppen der Reaktion durch 5 µl 1 M NaF (die Reaktion wird etwa 2 h verfolgt), erfolgt die Ablesung der Farbentwicklung, die zur Antikörpermenge proportional ist, durch ein Spektrophotometer.

Die Membranen von Tumorzellen haben einen anderen Glycoproteinbestand als die entsprechenden Normalzellen. Glycoproteine der Plasmamembranen werden an das Serum abgegeben

a) durch Sekretion solcher Glycoproteine, die vorübergehend Bestandteil von Plasmamembranen sind,

b) durch Abschilferung und

c) durch Zellalterung und Untergang.

Da es unwahrscheinlich ist, daß alle Hepatocarcinome ein gemeinsames spezifisches Glycoprotein exprimieren, das als Tumormarker benutzt werden könnte, wurde erfindungsgemäß mit Hilfe der monoklonalen Antikörper ein Glycoproteinmuster charakterisiert, das einer normalen Lebermembran entspricht. Dieses Muster wurde so gewählt, daß es durch eine maligne Transformation verändert wird. Im einzelnen wurden isolierte Membranglycoproteine aus der Leber (acht) und aus einem Hepatom (acht) untersucht. Der Nachweis eines veränderten Glycoproteinmusters im Patientenserum signalisierte eine maligne Transformation bzw. die Entstehung eines Hepatocarcinoms.

Bei der Herstellung von Antikörpern gegen Membranglycoproteine wurden bisher noch nie Antikörper getrennt gegen den Protein-bzw. Kohlenhydratanteil erzeugt und in Seren von Tumorpatienten getestet. Erfindungsgemäß wurden erstmals Antikörper sowohl gegen den Oligosaccharid- wie auch gegen den Proteinanteil erzeugt und in Tumorseren geprüft. Mit der Einbeziehung des Kohlenhydratanteils von Membranglycoproteinen als antigene Determinante erfährt die Tumordiagnostik eine bedeutsame molekulare Erweiterung.

Im einzelnen ist die Isolierung von je 8 Membranglycoproteinen aus der Leber und Morris-Hepatomen gelungen. Gegen diese Glycoproteine wurden mono-spezifische Antikörper erzeugt. Mit Hilfe der Immunpräzipitation wurde gezeigt, daß mindestens zwei der Glycoproteine (DPPIV), gp80) sowohl in der Leber als auch in Hepatomen vorkommen. Das gp80 wird von normalen Leberzellen an das Serum abgegeben, wobei sich seine Glycanstruktur im Serum von der des membranständigen Proteins unterscheidet. Noch ist nicht gesichert, ob und in welchen Konzentrationen das Hepatom gp80 ins Serum abgibt.

Auch das Vorkommen anderer Membranglycoproteine im Serum bzw. in Kulturüberständen primärer Hepatocyten konnte nachgewiesen werden. Weiterhin konnten mono-spezifische Antikörper hergestellt werden, die nur ein Glycoprotein der Hepatomembran ($M_r$ 175 k) erkennen. Dieses Glycorpotein ist besonders stark fucosyliert. Die Antikörper sind gegen diese ungewöhnliche Zuckerstruktur gerichtet.

Es wurde außerdem eine Reihe monoklonaler Antikörper zur Verfügung gestellt, die gegen Glycoproteine der Plasmamembran normaler Hepatocyten gerichtet sind.

| Monocl. Ak. | Antigen | Bemerkungen |
|---|---|---|
| 9,1 | 110, 135 kD | |
| 9,2 | 110 kD | Kommt nicht auf Hepatomen vor.. Ist an der gallekanalikulären Membran der Hepatocyten und zusätzlich im Dünndarm und Speicheldrüse lokalisiert. |
| 11,1 | 170 kD | Kommt nicht im Hepatom vor. |
| 11,3 | 130 kD | Kommt in hohen Konzentrationen in Leber- und Hepatomzellen vor. |
| 15,2 | 70 kD | Gegen alkalische Phosphatase gerichtet, kommt auch im Hepatom vor. |

Weitere Antikörper wurden bereits hergestellt; soweit die Antigene charakterisiert sind, fallen die enthaltenen Oligosaccharide alle unter die allgemeine Formel II.

Es wurde gefunden, daß in Hepatomen und Nierentumoren charakteristische Unterschiede der Oligosacchar-idstrukturen von Membranglycoproteinen gegenüber Normalgewebe bestehen. Auch gegen solche tumorspezifischen Oligosaccharide wurden poly- und monoklonale Antikörper hergestellt. Dazu werden Membranen von Tumoren isoliert, der Oligosaccharidanteil abgetrennt (enzymatisch und notfalls chemisch) und Antikörper gegen ihn induziert. Wegen einer wahrscheinlichen Kreuzreaktivität zwischen Mensch und Ratte und der leichteren Zugänglichkeit wurden zunächst Oligosaccharide aus Plasmamembranen von chemisch induzierten Hepatomen bei der Ratte untersucht und auf ihre Brauchbarkeit als Tumormarker geprüft. Es ist anzunehmen, daß sich die Untersuchungen auf menschliches Material übertragen lassen.

Außer gegen Hepatom-Oligosaccharide sind auch entsprechende Untersuchungen mit Oligosacchariden aus anderen Tumoren, insbesondere Dickdarmtumoren, durchgeführt worden.

Herstellung von Antikörpern

Isolierung von subzellulären Membranstrukturen aus Tumor-und Metastasengewebe durch Homogenisation des Gewebes in einem Puffer A, bestehend aus 10 mM $NaHCO_3$ und 0,5 mM $CaCl_2$, pH 7.0, und anschließenden Zentrifugationen. 1. Zentrifugation : 20 min bei 1.500 x g, Aufnahme des Sedimentes in Puffer A und anschließende Homogenisation im Ultraturrax oder mit dem Dounce-Homogenisator. 2. Zentrifugation wie oben. Das Sediment wird insgesamt 4 mal auf diese Weise gewaschen. Danach werden aus dem Sediment die Oligosaccharidstrukturen gewonnen. Dies geschieht entweder chemisch durch Hydrazinolyse oder enzymatisch durch Amidase F. Bei der chemischen Spaltung durch Hydrazinolyse der getrockneten (lyophilisierten) Sedimente (100°C für 10 h), indem zu 100 mg bis 1 g Membranfraktion 0,5 bis 5 ml frisch destilliertes, wasserfreies Hydrazin zugegeben und für 8-12 h bei 100 °C belassen werden (nach S. Takasaki, T. Mizouchi und

A. Kobata in "Methods in Enzymology" (1982) Vol. 83, 263-277). Anschließend wird das Reaktionsgemisch erhitzt (1 h) und zum Trocknen im Vakuum eingeengt (über konz. $H_2SO_4$ bei Raumtemperatur). Der Rückstand wird von Hydrazin durch wiederholte Evaporation mit Tuluol behandelt und dann in gesättigtem $NaHCO_3$ gelöst. Bei der enzymatischen Abspaltung der Oligosaccharidseitenketten mit Hilfe von Amidase F (E.C. 3.2.2.18) werden zu 10 bis 100 mg lyophilisierter Membranfraktion eine 1%ige Natriumdodecylsulfat (= SDS) in Wasser und Mercaptopropanol oder Mercaptoethanol (Endkonzentration 100 mM) zugesetzt und mit Ultraschall behandelt, für 1 min bei 100°C belassen, danach in 0,5 M Phosphatpuffer, pH 8.6, dem 1% Mega-10 (= Octanoyl-N-methylglucamid) aufgenommen und wieder mit Ultraschall behandelt. Durch Zusatz von Amidase F (12 mU/1 mg Protein, spaltet 1 nmol Oligosaccharid pro min ab) erfolgt die Abspaltung der Oligosaccharide (bei 41°C, 24 h). Die Auftrennung der Oligosaccharide wird an einer Säule, gefüllt mit BioGel P4, kleiner 0,04 mm (1,6 cm x 300 cm), durch Gelpermeationschromatographie vorgenommen und durch Anionenaustauschhochdruckflüssigkeitschromatographie an einer Säule, gefüllt mit Partisil AX-10, 2 x 4,6 x 250 mm, mit einem absteigenden Gradienten von $H_2O$/ 0,15 M $KH_2PO_4$, pH 4. Die Gelpermeationschromatographie erlaubt die Trennung nach der Anzahl n der Monosaccharide in einer Oligosaccharidfraktion (n = 1-22), die Anionen-HPLC erlaubt die Trennung einzelner Oligosaccharidfraktionen nach Ladungsunterschieden, die durch N-Acylneuraminsäuren (= Sialinsäuren) oder Sulfat bedingt sind. Um aus diesen Hapten Antigene herzustellen, werden noch die Oligosaccharide an Rinderserumalbumin oder Edestin (Methode von Kunz und Waldmann, Angewandte Chemie (1985) 24, 883-885) wie folgt gekoppelt: Das Oligosaccharid wird peracetyliert mittels Essigsäureanhydrid/Pyridin, in einer frischen kaltgesättigten Lösung von Chlorwasserstoff in Acetylchlorid suspendiert und 48 h bei Raumtemperatur gerührt. Nach Abdestillieren des Lösungsmittels wird der Rückstand aus Aceton/Ether umkristallisiert. Das so gewonnene Oligosaccharidchlorid wird in wasserfreiem Formamid unter Zugabe von Natriumazid (pro 1 Mol Oligosaccharid 20 Mole Azid) gelöst und 2 h bei 80°C gerührt. Das Reaktionsgemisch wird auf 100 ml $H_2O$ gegossen und die wäßrige Phase dreimal mit $CHCl_3$ extrahiert. Die vereinigten organischen Phasen werden über $MgSO_4$ getrocknet, eingeengt und der Rückstand aus Essigester/Ether umkristallisiert. Aus diesem Oligosaccharidazid wird das Oligosaccharidamin gewonnen durch Lösen dieses Azids in Ethanol und Zugabe von Platinoxidhydrat (pro mol Azid 100 mg Planinoxidhydrat).Nach 2 h bei Raumtemperatur wird der Ansatz abfiltriert und das Filtrat eingeengt. Zur Herstellung der vollständig geschützten Oligosaccharid-Asparagin-Albumin-Verbindung (peracetyliertes Oligosaccharid-$N^2$- (tert.-butyloxycarbonyl)-L-asparaginally-lester) wird das Oligosaccharidamin mit Boc-Asp-OAll (mol/mol) in Methylenchlorid gelöst und Ethyl-2-ethoxy-1, 2-dihydrochinolin-1-carboxylat (EEQD) im 1,5-fachen molaren Überschuß zugegeben. Nach 3 Tagen Rühren bei Raumtemperatur kristallisiert der Allylester aus. Die Verunreinigungen werden chromatographisch mit Essigester/Petrolether (1 : 1) abgetrennt und das Produkt mit Essigester eluiert. Die Spaltung des Allylesters erfolgt in wasser- und sauerstofffreiem Tetrahydrofuran und Dimethylsulfoxid durch Zugabe von Tetrakis(triphenylphosphin) palladium (o) unter Argon und Lichtabschluß. Danach wird Morpholin im 10-fachen molaren Überschuß zugetropft. Nach 4 h wird das Lösungsmittel im Hochvakuum abdestilliert und das Produkt chromatographisch gereinigt.

Gegen diejenigen Oligosaccharidfraktionen, die quantitativ in Tumoren nachweisbar sind, werden mono- und poly-klonale Antikörper hergestellt. Die Herstellung der monoklonalen Antikörper erfolgt nach der Methode von Köhler und Milstein ("Nature" (1975) Vol. 256, 495-497): Balb/c-Mäusen werden in Abständen von 10 Tagen 10-20 ug Antigen 3 bis 4 mal injiziert. Vier Tage nach der letzten (Booster)-Injektion werden den Mäusen die Milzen entnommen, die Milzzellen gewonnen und mit Myeloma-Zellen (NS-1) unter Zusatz von Polyethylenglykol (Endkonz. 38 %) fusioniert. Die positiven Zellklone werden rekloniert und die monoklonalen Antikörper aus dem Medium gewohnen. Die Reinigung der Antikörper erfolgt an einer Affi-Gel Blue-, gefolgt von einer HPLC-Säulenchromatographie (TSK- 3000 SW-Säule) nach Josic, Schütt, van Renswoude und Reutter ("J. Chromatogr." (1986) Vol. 353, 13-18) oder neuerdings an einer Protein A-Eupergit-Säule (Eupergit C30N der Fa. Röhm Pharma, Eupergit ist ein aktiviertes Polyacrylamid).

Die polyklonalen Antikörper werden durch Vermischen des gereinigten Antigens mit Öl zu einer micellaren Suspension und intracutane Injektion (10-20 quaddeln) in die Rückenhaut eines Kaninchens erzeugt. Die erste Injektion enthielt Freund'schges Adjuvans, die folgenden 2-4 Injektionen enthielten kein Adjuvans. Die Injektionen erfolgen in Abständen von 3 bis 4 Wochen. Das Blut (aus Ohrvene oder -arterie oder aus dem Herzen) wird zentrifugiert und aus der Serumfraktion in der oben beschriebenen Weise die Antikörper gewonnen.

## Patentansprüche

1. Antikörper, dadurch **gekennzeichnet**, daß sie Immunoglobuline der Klassen G und M mit Molekulargewichten von 100 000 bis 900 000 darstellen und gegen N-Glycanstrukturen von Zellmembranoberflächen gerichtet sind und im Immuntest (z.B. im RIA- und ELISA-Test oder im Mikrotiterplatten-Test) eine positive

Reaktion gegen antigene Oligosaccharide mit antigenen Strukturelementen aus Chitobiose-Mannose-Trisacchariden der allgemeinen Formel (I) zeigen:

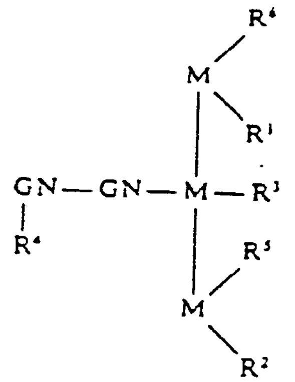

(I)

worin bedeuten:

| | |
|---|---|
| GN | N-Acetylglucosamin, |
| M | Mannose, wobei die Mannosereste über 1,3- und/oder 1,6-glycosidische Bindungen miteinander verknüpft sind und der Mannose-Rest am Chitobiose-Rest β-1,4-glycosidisch gebunden ist, |
| $R^1$, $R^2$, $R^5$ und $R^6$, | die gleich oder verschieden sind, jeweils Wasserstoff, den N-Acetylglucosamin-, Mannose-, Mannosedisaccharid- oder Lactosylamin-Rest GN-β-1,4-Gal, worin Gal für Galactose steht und GN und/oder Gal gegebenenfalls durch einen L-Fucosyl-Rest α-1,3-glycosidisch oder α-1,6-glycosidisch oder durch einen N-Acetylneuraminsäurerest vorzugsweise α-2,3-, α-2,6- oder α-2,8-glycosidisch substituiert ist (sind), |
| $R^3$ | Wasserstoff oder einen β-1,4-glycosidisch gebundenen und gegebenenfalls durch L-Fucose substituierten N-Acetylglucosamin- oder Lactosylamin-Rest GN-β-1,4-Gal, und |
| $R^4$ | Wasserstoff oder einen α-1,6-glycosidisch gebundenen L-Fucoserest. |

2. Antikörper nach Anspruch 1, dadurch gekennzeichnet, daß sie monoklonal erhältlich sind durch Koppeln eines Oligosaccharids mit einem Strukturelement aus Chitobiose-Mannose-Trisaccharid der Formel (I) als Hapten, Injizieren des Antigens bei einem Versuchstier, vorzugsweise Balb/c-Mäusen, Gewinnen der Milzzellen des immunisierten Tieres, Fusionieren der Milzzellen mit Myelom- oder Plasmocytom-Zellen und Gewinnen der Antikörper aus dem Medium der reklonierten positiven Klone.

3. Antikörper nach Anspruch 1, dadurch gekennzeichnet, daß sie monoklonal herstellbar sind durch Abspalten und Isolieren der Oligosaccharid-Fraktionen von Glycoproteinen aus Zellmembranoberflächen, Koppeln der Oligosaccharide als Haptene, Injizieren des Antigens bei einem Versuchstier, vorzugsweise Balb/c-Mäusen, Gewinnen der Milzzellen des immunisierten Tieres, Fusionieren der Milzzellen mit Myeloma- oder Plasmocytomzellen und Gewinnen der Antikörper aus dem Medium der reklonierten positiven Klone.

4. Antikörper nach Anspruch 1, dadurch gekennzeichnet, daß sie polyklonal herstellbar sind durch Abspalten und Isolieren der Oligosaccharid-Fraktionen von Glycoproteinen aus Zellmembranoberflächen, Koppeln der Oligosaccharide als Haptene, Injizieren einer Antigensuspension in die Rückenhaut eines Versuchstieres und Gewinnen der Antikörper aus der Serumfraktion des Blutes des Versuchstieres.

5. Antikörper nach Anspruch 3 oder 4, dadurch gekennzeichnet, daß die Oligosaccharid-Fraktion aus den Zellmembranoberflächen von Tumorzellen oder Metastasen derselben abgespalten wurden.

6. Antikörper nach einem der Ansprüche 1 bis 5, dadurch gekennzeichnet, daß es sich bei den antigenen Oligosacchariden um solche mit Strukturelementen der allgemeinen Formeln (II) bis (VI) handelt:

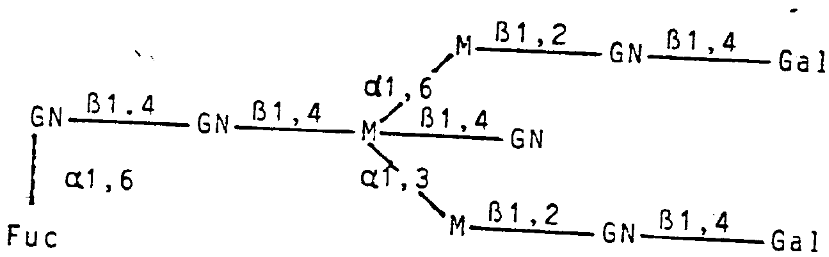

(II)

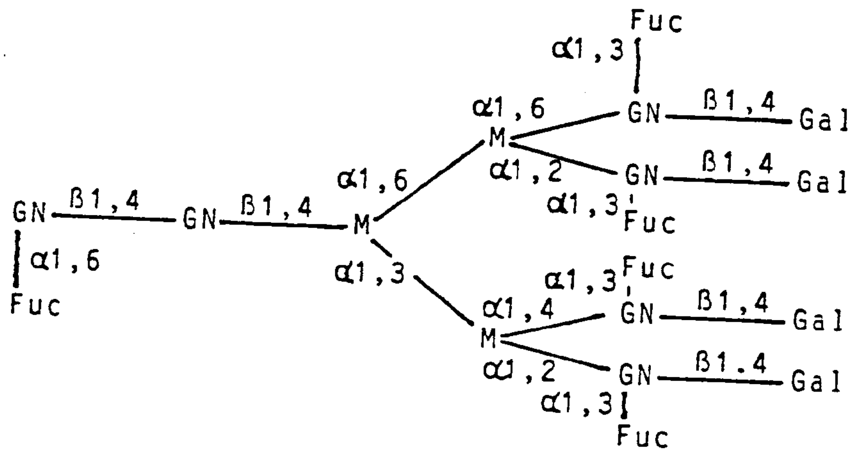

(III)

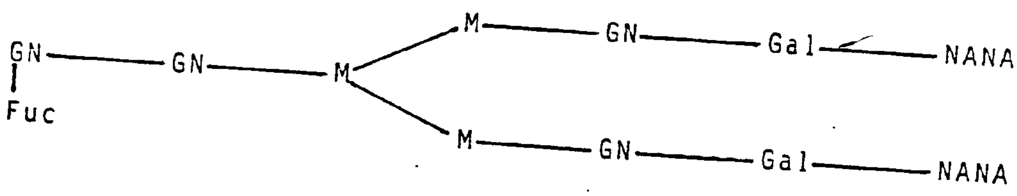

(IV)

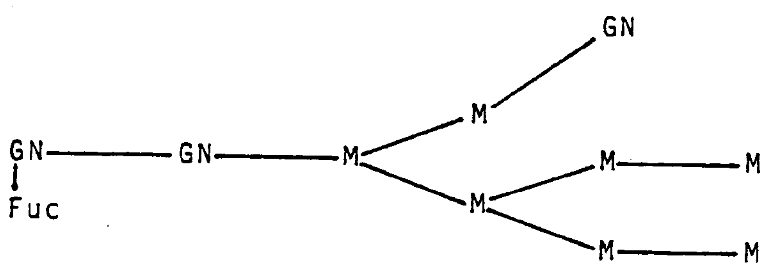

(V)

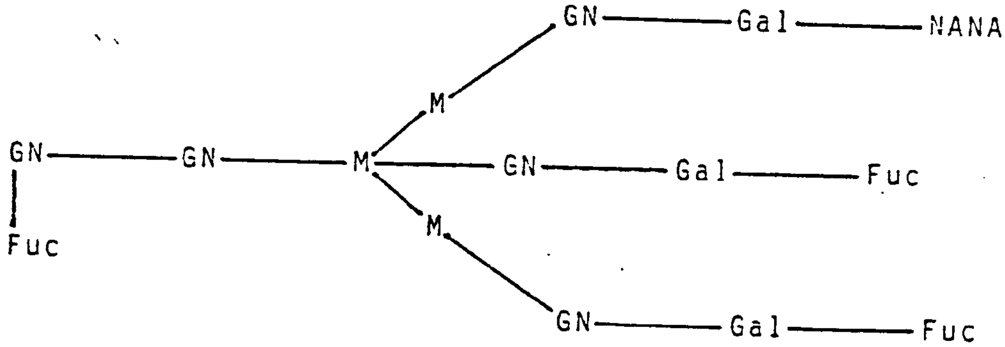

(VI)

worin GN, M und Gal die in Anspruch 1 angegebenen Bedeutungen haben und Fuc für L-Fucose und NANA für N-Acetylneuraminsäure stehen.

7. Verfahren zur Herstellung der Antikörper nach den Ansprüchen 1 bis 7, dadurch gekennzeichnet, daß man die Oligosaccharid-Fraktionen aus den Glycoproteinen der Zellmembranoberflächen abtrennt und immunologisch zur Erzeugung der Antikörper verwendet.

8. Verfahren nach Anspruch 7, dadurch gekennzeichnet, daß man die Oligosaccharid-Fraktionen aus den Glycoproteinen der Zellmembranoberflächen abspaltet und isoliert, die Oligosaccharide als Haptene koppelt, die Antigene einem Versuchstier, vorzugsweise Balb/c-Mäusen, injiziert, die Milzzellen des immunisierten Versuchstiers gewinnt und mit Myelom- oder Plasmacytom-Zellen fusioniert und die Antikörper aus dem Medium der reklonierten positiven Klone gewinnt.

9. Verfahren nach Anspruch 7 oder 8, dadurch gekennzeichnet, daß man die Oligosaccharid-Fraktionen aus den Glycoproteinen der Zellmembranoberflächen abspaltet und isoliert und die Oligosaccharide als Haptene koppelt, eine Suspension der Antigene in die Rückenhaut eines Versuchstieres injiziert und die Antikörper aus der Serumfraktion des Blutes des Versuchstieres gewinnt.

10. Verfahren nach einem der Ansprüche 7 bis 9, dadurch gekennzeichnet, daß die Oligosaccharid-Fraktion durch Hydrazinolyse chemisch abgespalten wird.

11. Verfahren nach einem der Ansprüche 7 bis 10, dadurch gekennzeichnet, daß die Oligosaccharid-Fraktion enzymatisch abgespalten wird.

12. Verfahren nach einem der Ansprüche 7 bis 11, dadurch gekennzeichnet, daß die Oligosaccharid-Fraktion aus Glycoproteinen von Tumor- oder Metastasezellen abgespalten wird.

13. Verfahren nach einem der Ansprüche 7 bis 12, dadurch gekennzeichnet, daß die Haptene mit Rinderserumalbumin oder Edestin gekoppelt werden.

14. Klon zur Erzeugung von Antikörpern gegen N-Glycanstrukturen von Zellmembranoberflächen nach den Ansprüchen 1 bis 13, dadurch gekennzeichnet, daß er durch Fusionieren von Myelom- oder Plasmocytom-Zellen mit den Milzzellen eines Versuchstieres herstellbar ist, das mit Oligosaccharid-Antigenen immunisiert wurde, die aus der Oligosaccharid-Fraktion von Glycoproteinen der Zellmembranoberflächen gebildet wurden.

15. Verwendung der Antikörper nach den Ansprüchen 1 bis 13 zur Herstellung eines Mittels für die Diagnose und Therapie von Tumoren, insbesondere Hepatomen und Dickdarmtumoren.

## Revendications

1. Anticorps caractérisés en ce qu'ils représentent des immunoglobulines des classes G et M ayant des poids moléculaires de 100.000 à 900.000, en ce qu'ils sont dirigés contre des structures N-glucaniques de la surface de membranes cellulaires et en ce qu'ils manifestent, dans le test immunologique (par exemple, dans le dosage RIA et dans le dosage ELISA ou encore dans l'essai à l'aide de plaques microtitre), une réaction positive contre des oligosaccharides antigènes comprenant des éléments de structure antigènes de chitobiose-mannose-trisaccharides répondant à la formule générale (I) :

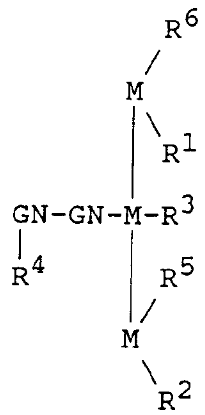

(I)

dans laquelle

| | |
|---|---|
| GN | représente la N-acétylglucosamine, |
| M | représente le mannose, les restes de mannose étant reliés l'un à l'autre par des liaisons 1,3- et/ou 1,6-glucosidiques et le reste de mannose étant lié au reste de chitobiose par une liaison β-1,4-glucosidique, |
| $R^1$, $R^2$, $R^5$ et $R^6$ | qui sont identiques ou différents représentent chacun un atome d'hydrogène, le reste de N-acétylglucosamine, de mannose, de mannose disaccharide ou de lactosylamine GN-β-1,4-Gal dans lequel Gal représente le galactose et GN et/ou Gal peuvent éventuellement être substitués par un reste L-fucosyle par une liaison α-1,3-glucosidique ou α-1,6-glucosidique, ou par un reste d'acide N-acétylneuraminique de préférence par une liaison α-2,3-, α-2,6- ou α-2,8-glucosidique, |
| $R^3$ | représente un atome d'hydrogène ou un reste de N-acétylglucosamine ou de lactosylamine GN-β-1,4-Gal à liaison β-1,4-glucosidique et éventuellement substitué par un reste L-fucose, et |
| $R^4$ | représente un atome d'hydrogène ou un reste L-fucose à liaison α-1,6-glucosidique. |

2. Anticorps selon la revendication 1, caractérisés en ce qu'on peut les obtenir par voie monoclonale, par couplage d'un oligosaccharide avec un élément de structure de chitobiose-mannose-trisaccharide répondant à la formule (I), sous forme de haptène, par injection de l'antigène à un animal d'expérience, de préférence des souris Balb/c, par récupération des cellules spléniques de l'animal immunisé, par fusion des cellules spléniques avec des cellules de myélome ou de plasmocytome et par récupération des anticorps hors du milieu des clones positifs reclonés.

3. Anticorps selon la revendication 1, caractérisés en ce qu'on peut les préparer par voie monoclonale par séparation et isolation des fractions d'oligosaccharides de glycoprotéines à partir de la surface de membranes cellulaires, par couplage des oligosaccharides sous forme de haptènes, par injection de l'antigène à un animal d'expérience, de préférence des souris Balb/c, par récupération des cellules spléniques de l'animal immunisé, par fusion des cellules spléniques avec des cellules de myélome ou de plasmocytome et par récupération des anticorps hors du milieu des clones positifs reclonés.

4. Anticorps selon la revendication 1, caractérisés en ce qu'on peut les préparer par voie polyclonale par séparation et isolation des fractions d'oligosaccharides de glycoprotéines à partir de la surface de membranes cellulaires, par couplage des oligosaccharides sous forme de haptènes, par injection d'une suspension d'antigènes dans la peau dorsale d'un animal d'expérience et par récupération des anticorps hors de la fraction sérique du sang de l'animal d'expérience.

5. Anticorps selon la revendication 3 ou 4, caractérisés en ce que la fraction d'oligosaccharides a été séparée de la surface des membranes cellulaires de cellules tumorales ou de métastases de ces dernières.

6. Anticorps selon l'une quelconque des revendications 1 à 5, caractérisés en ce que, quant aux oligosaccharides antigènes, il s'agit de ceux possédant des éléments de structure répondant aux formules générales (II) à (VI) :

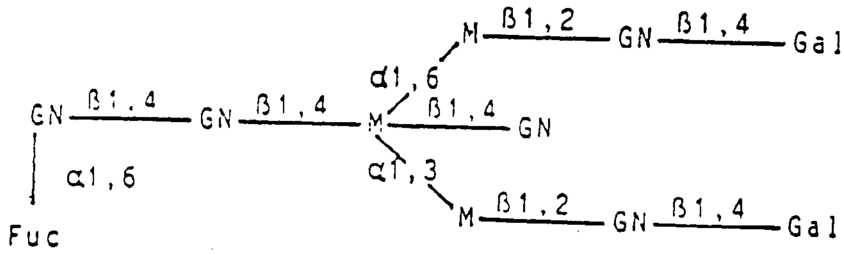

(II)

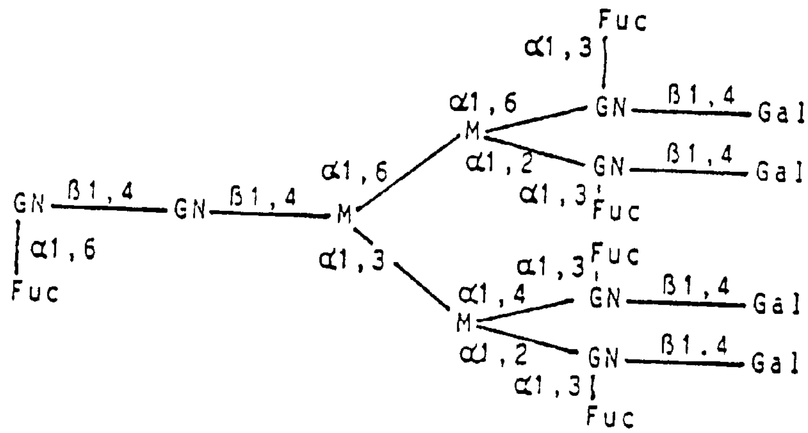

(III)

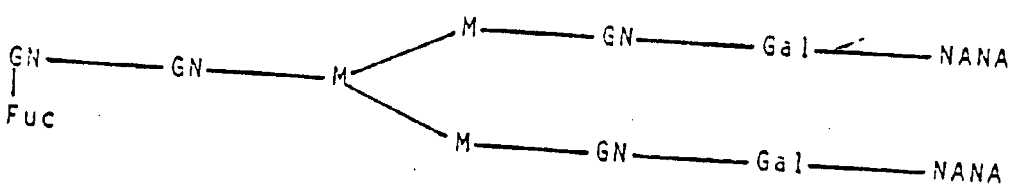

(IV)

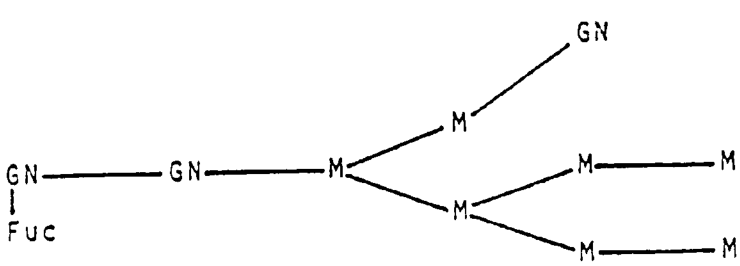

(V)

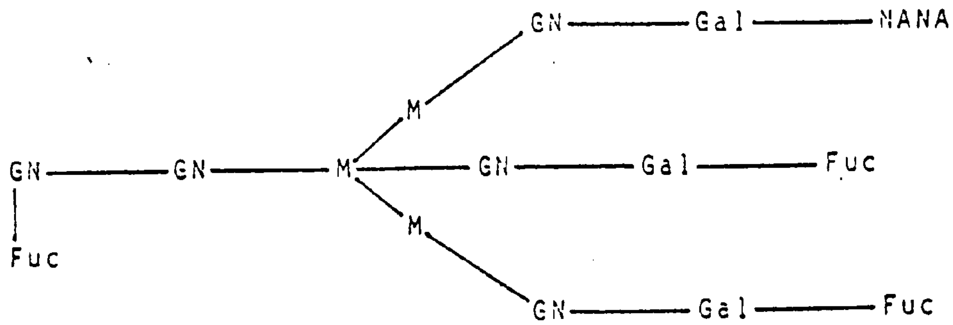

(VI)

dans lesquelles GN, M et Gal ont les significations indiquées à la revendication 1 et Fuc représente le L-fucose et NANA représente l'acide N-acétylneuraminique.

7. Procédé pour la préparation des anticorps selon les revendications 1 à 7, caractérisé en ce qu'on sépare les fractions d'oligosaccharides à partir des glycoprotéines de la surface des membranes cellulaires et on les utilise par voie immunologique pour la production des anticorps.

8. Procédé selon la revendication 7, caractérisé en ce qu'on sépare et on isole les fractions d'oligosaccharides à partir des glycoprotéines de la surface des membranes cellulaires, on couple les oligosaccharides sous forme de haptènes, on injecte les antigènes à un animal d'expérience, de préférence des souris Balb/c, on récupère les cellules spléniques de l'animal d'expérience immunisé et on les fusionne à des cellules de myélome ou de plasmocytome et on récupère les anticorps à partir du milieu des clones positifs reclonés.

9. Procédé selon la revendication 7 ou 8, caractérisé en ce qu'on sépare et on isole les fractions d'oligosaccharides des glycoprotéines de la surface des membranes cellulaires, on couple les oligosaccharides sous forme de haptènes, on injecte une suspension des antigènes dans la peau dorsale d'un animal d'expérience et on récupère les anticorps hors de la fraction sérique du sang de l'animal d'expérience.

10. Procédé selon l'une quelconque des revendications 7 à 9, caractérisé en ce qu'on sépare la fraction d'oligosaccharides par voie chimique par hydrazinolyse.

11. Procédé selon l'une quelconque des revendications 7 à 10, caractérisé en ce qu'on sépare la fraction d'oligosaccharides par voie enzymatique.

12. Procédé selon l'une quelconque des revendications 7 à 11, caractérisé en ce qu'on sépare la fraction d'oligosaccharides de glycoprotéines de cellules tumorales ou métastatiques.

13. Procédé selon l'une quelconque des revendications 7 à 12, caractérisé en ce qu'on couple les haptènes à de l'albumine de sérum bovin ou à de l'édestine.

14. Clone pour la production d'anticorps contre des structures N-glucaniques de la surface de membranes cellulaires selon les revendications 1 à 13, caractérisé en ce qu'on peut le préparer par fusion de cellules de myélome ou de plasmocytome avec les cellules spléniques d'un animal d'expérience qui a été immunisé avec des antigènes d'oligosaccharides qui ont été formés à partir de la fraction d'oligosaccharides de glycoprotéines de la surface des membranes cellulaires.

15. Utilisation des anticorps selon les revendications 1 à 13, pour la préparation d'un agent pour le diagnostic et la thérapie de tumeurs, en particulier d'hépatomes et de tumeurs du côlon.

## Claims

1. Antibodies, characterised in that they are class G and M immunoglobulins having molecular weights of 100 000 to 900 000 and are specific for N-glycan structures of cell membrane surfaces and which, in an

immunoassay (eg. RIA and ELISA assay or microtiter plates) exhibit a positive reaction to antigenic oligosaccharides with antigenic structural elements of chitobiose and mannosetrisaccharides of the general formula (I):

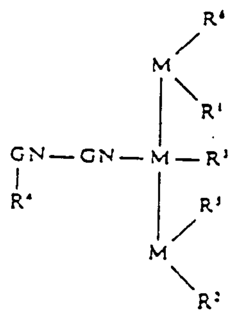

(I)

in which:
GN denotes N-acetylglucosamine;
M denotes mannose, wherein the mannose residues are linked to each other through 1,3- and/or 1,6-glycosidic linkages and the mannose residue is linked β-1,4-glycosidically to the chitobiose residue;
$R^1$, $R^2$, $R^5$ and $R^6$, which may be identical or different, in each case denote hydrogen, a N-acetylglucosamine, mannose, mannose-disaccharide or lactosylamine residue GN-β-1,4-Gal, wherein Gal stands for galactose and GN and/or animal is/are in each case optionally substituted α-1,3-glycosidically or α-1,6-glycosidically by an L-fucosyl residue or preferably α-2,3-, α-2,6- or α-2,8-glycosidically by a N-acetylneuroaminic acid residue;
$R^3$ denotes hydrogen or a N-acetylglucosamine or lactosylamine residue GN-β-1,4-Gal which is β-1,4-glycosidically linked and optionally substituted by L-fucose; and
$R^4$ denotes hydrogen or an α-1,6-glycosidically linked L-fucose residue.

2. Antibodies according to Claim 1, characterised in that they can be obtained monoclonally as a result of an oligosaccharide being coupled to a structural element of chitobiose and mannose trisaccharides of formula (I) as hapten, the antigen being injected into a test animal, preferably Balb/c-mice, the spleen cells being extracted from the immunized animal, the spleen cells being fused with myeloma or plasmocytoma cells and the antibodies being extracted from the medium of the recloned positive clones.

3. Antibodies according to Claim 1, characterised in that they can be produced monoclonally as a result of the oligosaccharide fractions being separated and isolated from glycoproteins from cell membrane surfaces, the oligosaccharides being coupled as haptens, the antigen being injected into a test animal, preferably Balb/c-mice, the spleen cells being extracted from the immunized animal, the spleen cells being fused with myeloma or plasmocytoma cells and the antibodies being extracted from the medium of the recloned positive clones.

4. Antibodies according to Claim 1, characterised in that they can be produced polyclonally as a result of the oligosaccharide fractions being separated and isolated from glycoproteins from cell membrane surfaces, the oligosaccharides being coupled as haptens, an antigen suspension being injected into the skin on the back of a test animal, and the antibodies being extracted from the serum fraction of the blood of the test animal.

5. Antibodies according to Claim 3 or 4, characterised in that the oligosaccharide fraction has been separated from the cell membrane surfaces of tumour cells or metastases thereof.

6. Antibodies according to any one of Claims 1 to 5, characterised in that the antigenic oligosaccharides are those having structure elements of the general formulae (II) to (VI):

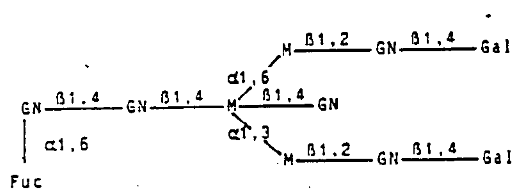

(II)

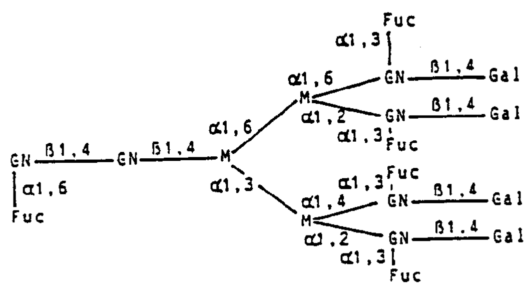

(III)

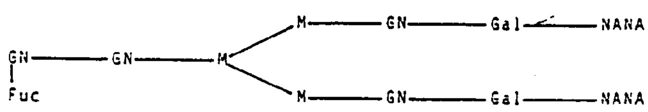

(IV)

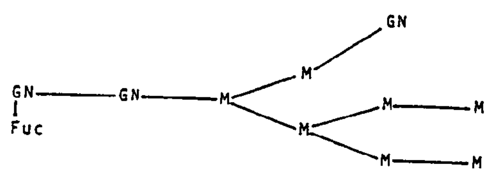

(V)

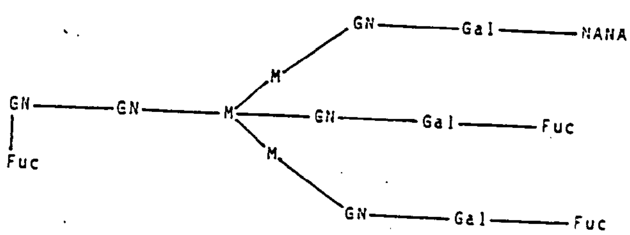

(VI)

in which GN, M and Gal have the meanings given in Claim 1, Fuc denotes L-fucose and NANA denotes N-acetylneuroaminic acid.

7. Process for producing the antibodies according to Claims 1 to 7, characterised in that the oligosaccharide fractions are separated from the glycoproteins of the cell membrane surfaces and are used immunologically to produce the antibodies.

8. Process according to Claim 7, characterised in that the oligosaccharide fractions are separated and isolated from the glycoproteins of the cell membrane surfaces, the oligosaccharides are coupled as haptens, the antigens are injected into a test animal, preferably Balb/c-mice, the spleen cells of the immunized test animal are extracted and fused with myeloma or plasmocytoma cells, and the antibodies are extracted from the medium of the recloned positive clones.

**9.** Process according to either of Claims 7 or 8, characterised in that the oligosaccharide fractions are separated and isolated from the glycoproteins of the cell membrane surfaces, and the oligosaccharides are coupled as haptens, a suspension of the antigens is injected into the skin on the back of a test animal, and the antigens are extracted from the serum fraction of the blood of the test animal.

**10.** Process according to any one of Claims 7 to 9, characterised in that the oligosaccharide fraction is chemically separated by hydrazinolysis.

**11.** Process according to any one of Claims 7 to 10, characterised in that the oligosaccharide fraction is separated enzymatically.

**12.** Process according to any one of Claims 7 to 11, characterised in that the oligosaccharide fraction is separated from glycoproteins of tumour or metastases cells.

**13.** Process according to any one of Claims 7 to 12, characterised in that the haptens are coupled with bovine serum albumin or edestin.

**14.** Clone for producing antibodies to N-glycane structures of cell membrane surfaces according to any one of Claims 1 to 13, characterised in that it can be produced as a result of myeloma or plasmocytoma cells being fused with the spleen cells of a test animal which has been immunized with oligosaccharide antigens produced from the oligosaccharide fraction of glycoproteins of the cell membrane surfaces.

**15.** Use of the antibodies according to any one of Claims 1 to 13 for producing an agent for diagnosing and treating tumours, in particular hepatomae and tumours of the colon.